# EUROPEAN PATENT SPECIFICATION

(11) **EP 4 112 047 B1**
(45) Date of publication and mention of the grant of the patent: **20.08.2025**
(21) Application number: 21779431.2
(22) Date of filing: 12.03.2021
(51) Int. Cl.: A61K 9/24, A61K 9/20, A61K 31/4985, A61K 31/7034, A61K 31/155, A61K 31/70

(54) **ORAL COMPLEX TABLET COMPRISING SITAGLIPTIN, DAPAGLIFLOZIN, AND METFORMIN**
ORALE KOMPLEXE TABLETTE MIT SITAGLIPTIN, DAPAGLIFLOZIN UND METFORMIN
COMPRIMÉ ORAL COMPLEXE COMPRENANT DE LA SITAGLIPTINE, DE LA DAPAGLIFLOZINE ET DE LA METFORMINE

(30) Priority: 30.03.2020 KR 20200038568
(43) Date of publication of application: 04.01.2023
(73) Proprietor: Hanmi Pharm. Co., Ltd., Hwaseong-si, Gyeonggi-do 18536 (KR)
(72) Inventor: TAK, Jin Wook, Hwaseong-si, Gyeonggi-do 18477 (KR); KIM, Bo Sik, Seoul 08807 (KR); KWON, Taek Kwan, Hwaseong-si, Gyeonggi-do 18466 (KR); IM, Ho Taek, Yongin-si, Gyeonggi-do 16909 (KR); KIM, Yong Il, Gwacheon-si, Gyeonggi-do 13835 (KR)
(74) Representative: Brevalex
(86) International application number: PCT/KR2021/003080
(87) International publication number: WO 2021/201461

(56) References cited:
- EP-B1- 1 948 149
- WO-A1-2013/110085
- WO-A1-2018/124497
- KR-A- 20160 111 237
- KR-A- 20190 038 283
- KR-A- 20190 130 432
- US-A1- 2015 238 421

## Description

### TECHNICAL FIELD

The present disclosure relates to a composite tablet for oral administration, including sitagliptin, dapagliflozin, and metformin as active ingredients, and more particularly to a composite tablet for oral administration having excellent storage stability and high productivity, and a method of preparing the same.

### BACKGROUND ART

In general, type 2 diabetes patients are accompanied by being overweight, abdominal obesity, and high blood pressure, and thus diabetes is known as a disease that causes secondary chronic diseases or metabolic syndromes, such as hypertension, hyperlipidemia, myocardial infarction, and stroke. According to the medical guidelines of the Korean Diabetes Association, combined drug therapy is actively recommended to enhance improvement of symptoms. In particular, the combined use of DPP-4 inhibitor drugs and SGLT-2 inhibitor drugs has recently been proven by academia to have excellent efficacy in the treatment of diabetes, and even three-drug treatment with metformin is also under research.

Sitagliptin (Product name: JANUVIA tablet) is a dipeptidyl peptidase-4 (DPP-4) inhibitor drug and its compound name is (R)-3-amino-1-(3-(trifluoromethyl)-5,6-dihydro-[1,2,4]triazolo[4,3-a]pyrazin-7(8H)-yl)-4-(2,4,5-trifluorophenyl)butan-1-one. Sitagliptin regulates blood sugar by inhibiting the breakdown of gastrointestinal hormones called incretins to enable the incretins, which regulate insulin and glucagon, to function well in the body. It is known that when sitagliptin is orally administered to a patient with type 2 diabetes, HbA1c levels are significantly reduced, and fasting blood sugar and postprandial blood sugar secretion are reduced.

Dapagliflozin (Product name: FORXIGA tablet) is a sodium-glucose linked transporter 2 (SGLT-2) inhibitor drug, and its compound name is (2S,3R,4R,5S,6R)-2-[4-chloro-3-(4-ethoxybenzyl)phenyl]-6-(hydroxymethyl)tetrahydro-2H-pyran-3,4,5-triol.
Dapagliflozin selectively inhibits SGLT2 in the kidneys and increases the excretion of glucose in the urine, thereby improving insulin sensitivity and delaying the onset of diabetic complications, and thus allowing plasma glucose levels to be normalized. Dapagliflozin is currently sold on the market by the original developer AstraZeneca AB, in the form of tablets (FORXIGA tablet) including dapagliflozin propylene glycol hydrate as an active ingredient.

Metformin is an oral antihyperglycemic drug mainly used for the treatment of type 2 diabetes patients as a biguanide-based therapeutic agent for diabetes. The glycemic control mechanism of metformin functions independently of insulin secretion, and for example, it is known that metformin activates glucose transporters in the liver. Metformin induces weight loss in diabetic patients, and has the effect of decreasing blood triglycerides and low-density lipoproteins and increasing high-density lipoproteins. Therefore, metformin may be used as a primary drug for non-insulin-dependent diabetes patients who have insulin resistance.

Metformin, as its hydrochloride salt, is commercially available in tablet form as Glucophage (Bristol-Myers Squibb Company). The commercially available Glucophage tablets contain 500 mg, 850 mg, or 1,000 mg of metformin hydrochloride salt, and the administration thereof is carried out within a range that does not exceed the maximum required dose of 2,550 mg per day in consideration of both efficacy and tolerance. Side effects associated with the use of metformin include loss of appetite, bloating, nausea, and diarrhea, which appear in 20 percent (%) to 30 % of patients taking metformin. The side effects are transient and most often disappear 2 to 3 weeks after taking metformin. If diarrhea or severe abdominal bloating continues, it is advisable to stop administration of metformin. Rarely, skin rashes and hives may occur. These side effects may be partially avoided by reducing the minimum and/or sustained dose or by using sustained-release formulations that allow the dosing frequency to be reduced.

In diabetes, one or more antidiabetic drugs are often administered in combination for the purpose of controlling blood sugar and reducing side effects. Sitagliptin and dapagliflozin have the main effect of blood sugar reduction without a risk of low blood sugar. In addition, sitagliptin has the effects of protecting pancreatic beta cells and increasing GLP-1, and dapagliflozin has the effects of weight loss and blood pressure reduction, and has also been introduced with clinical results wherein a combination of two active ingredients has a synergistic effect. In addition, it has been reported that, when sitagliptin alone or dual administration of sitagliptin and metformin is not effective in glycemic control, triple administration of sitagliptin, metformin, and dapagliflozin by adding dapagliflozin may be effective in glycemic control (Diabetes Care 2014 Mar; 37(3): 740-750). Patent document US 2015/0238421 discloses bilayer tablets comprising metformin hydrochloride and dapagliflozin.

In addition, in the case of diabetic patients, as diabetes progresses, it becomes difficult to control blood sugar, resulting in complications. Specifically, elderly diabetic patients are more likely to suffer from high blood pressure, obesity, and hyperlipidemia. Due to these characteristics of diabetic patients, medication compliance is a very crucial factor, a reduction in medication compliance not only lowers a patient's quality of life, but also reduces a patient's treatment rate, increasing personal medical expenses and worsening insurance finances. Therefore, it is necessary to develop a triple composite tablet containing all of sitagliptin, dapagliflozin, and metformin as active ingredients.

However, the development of such a triple composite tablet is difficult due to several problems such as productivity and stability of tablets due to differences in physical properties of each active pharmaceutical ingredient (API). For example, metformin requires wet-granulation due to problems such as flowability during tablet manufacturing, whereas sitagliptin and dapagliflozin are unstable to water. In addition, the productivity of dapagliflozin is not satisfactory because the volume of the active ingredient is large in spite of a small amount due to its low density, and layer separation with other active ingredients and excipients may occur.

### [Prior Art Document]

### [Non-patent Document]

1. Diabetes Care 2014 Mar; 37(3): 740-750

### DESCRIPTION OF EMBODIMENTS

### TECHNICAL PROBLEM

According to an aspect, provided is a triple composite tablet including sitagliptin, dapagliflozin, and metformin and having excellent storage stability and excellent productivity.

According to another aspect, provided is a method of preparing the composite tablet.

Other objects and advantages of the present application will become more apparent from the following detailed description in conjunction with the appended claims. Contents not described in this specification can be sufficiently recognized and inferred by a person skilled in the art within the technical field of the present application or a similar technical field, and thus description thereof is omitted.

### SOLUTION TO PROBLEM

According to an aspect, provided is a composite tablet including:
a first layer comprising dry granules prepared by dry-granulation that include sitagliptin or a pharmaceutically acceptable salt thereof, or a hydrate thereof, and
dapagliflozin or a pharmaceutically acceptable salt thereof, or a hydrate thereof; and
a second layer comprising wet granules prepared by wet-granulation that include metformin or a pharmaceutically acceptable salt thereof and colloidal silicon dioxide, wherein the colloidal silicon dioxide is present at 0.7 percent by weight (wt%) to 2.8 wt% relative to active ingredients of metformin.

According to another aspect, provided is a method of preparing the composite tablet for oral administration according to the one aspect, the method including:
preparing a mixture portion including sitagliptin or a pharmaceutically acceptable salt thereof, or a hydrate thereof, and dapagliflozin or a pharmaceutically acceptable salt thereof, or a hydrate thereof, and an excipient;
dry-granulating the mixture portion;
preparing a first mixture portion by adding a lubricant to the obtained granules and mixing the lubricant with the granules;
preparing metformin wet granules containing metformin or a pharmaceutically acceptable salt thereof, and an excipient;
drying the obtained metformin wet granules;
preparing a second mixture portion by mixing the dried metformin wet granules with colloidal silicon dioxide and a lubricant; and
compressing the first mixture portion into a first layer and the second mixture portion into a second layer, by using a double-layer tablet press.

### ADVANTAGEOUS EFFECTS OF DISCLOSURE

According to an aspect, the stability of the active ingredients may be secured while a formulation includes a first layer containing sitagliptin and dapagliflozin and a second layer containing metformin, and tablets with excellent productivity may be obtained because tableting failures such as capping or laminating do not occur.

### BRIEF DESCRIPTION OF DRAWINGS

FIG. 1 shows images of double-layer tablets according to an embodiment, prepared by varying the water content in metformin wet granules when preparing the double-layer tablets;
FIG. 2 is a graph showing required compression pressure (y-axis) according to water content of metformin granules (x-axis) measured from double-layer tablets including various contents of colloidal silicon dioxide and various water contents of metformin granules, according to an embodiment;
FIG. 3 is a graph showing differences in shrinkage of double-layer tablets (y-axis) according to colloidal silicon dioxide content included in the second layer (x-axis), measured from double-layer tablets including various contents of colloidal silicon dioxide and various water contents of metformin granule layers, according to an embodiment;
FIG. 4 is an image of a tablet according to an embodiment;
FIG. 5 is a graph showing measurement results of the total amount of related substances of sitagliptin measured over time from double-layer tablets, including colloidal silicon dioxide of various contents and metformin granule layers of various water contents under harsh conditions of 60 °C, according to an embodiment; and
FIG. 6 is a graph showing measurement results of the total amount of related substances of dapagliflozin, measured over time from double-layer tablets including various contents of colloidal silicon dioxide and various water contents of metformin granule layers under harsh conditions of 60 °C.

### MODE OF DISCLOSURE

Hereinafter, the present disclosure will be described in more detail.

All technical terms used herein have the same meaning as commonly understood by one of ordinary skill in the art, unless defined otherwise. As used herein, the term "about" means that the referenced value may vary to some extent. For example, the value may vary by 10%, 5%, 2%, or 1% For example, "about 5" is meant to include any value between 4.5 and 5.5, between 4.75 and 5.25, or between 4.9 and 5.1, or between 4.95 and 5.05. As used herein, the terms "has", "may have", "comprises", or "may include" indicate the presence of a corresponding feature (e.g., a numerical value or a component such as an ingredient), and does not exclude the presence of additional features.

As used herein, the term "shrinkage difference of double-layer tablet" refers to the difference in the shrinkage of each layer of the double-layer tablet. The shrinkage of each layer was measured by measuring a difference between a diameter of a major axis of a tablet immediately after tableting and the diameter of the major axis of the tablet after storage at 40 °C for 1 hour, and then calculating the ratio of the difference to the diameter of the major axis of the tablet immediately after tableting as a percentage.

The term "water content in wet granules of metformin" refers to the weight of water in percentage (%) relative to the weight of wet granules when the wet granules are dried after preparation.

The term "required compression pressure" refers to a compression pressure required for tableting in the tablet manufacturing process to have a desired tablet hardness.

According to an aspect, provided is a composite tablet including:
a first layer including dry granules that includes sitagliptin or a pharmaceutically acceptable salt thereof, or a hydrate thereof, and
dapagliflozin or a pharmaceutically acceptable salt thereof, or a hydrate thereof; and
a second layer containing wet granules that includes metformin or a pharmaceutically acceptable salt thereof and colloidal silicon dioxide.

The active ingredient sitagliptin and dapagliflozin may include all of the crystalline forms, hydrates, co-crystals, solvates, salts, diastereomers, or enantiomers thereof.

Metformin, the active ingredient of the first layer, may include all of the crystalline forms, co-crystals, solvates, or isomers.

The pharmaceutically acceptable salt thereof refers to any pharmaceutically acceptable salt that may be commonly used in the art.

In one embodiment, the sitagliptin or a pharmaceutically acceptable salt thereof, or a hydrate thereof, may be sitagliptin phosphate hydrate.

In one embodiment, the dapagliflozin or a pharmaceutically acceptable salt thereof, or a hydrate thereof may be a pharmaceutically acceptable co-crystal of dapagliflozin. In one embodiment, the dapagliflozin or a pharmaceutically acceptable salt thereof may be dapagliflozin L-proline or dapagliflozin propandiol hydrate. In one embodiment, the dapagliflozin or a pharmaceutically acceptable salt thereof may be dapagliflozin L-proline.

In one embodiment, the metformin may be a metformin hydrochloride salt or a metformin free base.

As used herein, sitagliptin, dapagliflozin, and metformin may be construed as including all of the salts, solvates, and isomers thereof.

The composite tablet according to an aspect may include a first layer including dry granules containing sitagliptin and dapagliflozin and a second layer including wet granules that may include metformin and colloidal silicon dioxide. Since sitagliptin and dapagliflozin are relatively vulnerable to moisture relative to metformin, the stability of the active ingredients may be promoted by having a double-layer tablet structure as described above. In addition, metformin has low flowability and has a form of wet granules, thereby securing excellent flowability and high productivity upon manufacturing the double-layer tablet.

In one embodiment, a shrinkage difference between the first layer and the second layer may be within 1 percent (%). When the shrinkage difference exceeds 1 %, layer separation between the first layer and the second layer may occur during storage (see Experimental Example 1).

According to the claims, in the second layer, the colloidal silicon dioxide may be present at 0.7 percent by weight (wt%) to 2.8 wt% relative to active ingredients of metformin.

In one embodiment, the metformin wet granules of the second layer have a water content of 2.5 wt% to 3.5 wt%.

In one embodiment, the colloidal silicon dioxide is present at 0.7 wt% to 2.8 wt% relative to active ingredients of metformin, and the metformin wet granules of the second layer have a water content of 2.5 wt% to 3.5 wt%

As the second layer contains colloidal silicon dioxide, a composite tablet with appropriate hardness (hardness range including 20 kp) may be prepared. As a result of the experiment, it was found that inclusion of colloidal silicon dioxide may lower the compression pressure during tableting to ensure that the composite tablet has an appropriate hardness. When the compression pressure is high during tableting, tableting failures such as capping or laminating of tablets may occur. However, by containing colloidal silicon dioxide, it was confirmed that the compression pressure may be lowered, and thus, a double-layer tablet having an appropriate hardness may be manufactured without tableting failures (see Experimental Example 2). In addition, as a result of the experiment, when the content of the colloidal silicon dioxide was more than 2.8 wt% relative to the active ingredient of metformin, it showed an increase in the related substance exceeding the standard or close to the standard for both sitagliptin and dapagliflozin in 4 weeks under harsh conditions. Also, overall, as the amount of colloidal silicon dioxide increased, the related substances of sitagliptin and dapagliflozin increased. Therefore, it was evaluated that the amount of colloidal silicon dioxide used within 2.8 wt% of the active ingredient of metformin may ensure stability (Experimental Example 3).

It was confirmed that metformin wet granules of the second layer affected the productivity and shrinkage difference between layers according to the water content. When metformin wet granules contained more than 3.5 % of water, tableting failure did not occur during tableting of double-layer tablets. However, when coating the tablets and under accelerated and harsh conditions, the shrinkage difference between the first layer and the second layer exceeded about 1 %, inducing layer separation occurred. In addition, when metformin wet granules contained less than 2.0 % of water, it was found that the hardness of the tablets was not ensured and easily broken during tableting due to insufficient water (see Experimental Example 1). In addition, it was found that as the water content of metformin wet granules increased, the compression pressure required for the composite tablet to have a desired hardness, i.e., the required compression pressure, may be lowered. When the compression pressure is high during tableting, tableting failures such as capping or laminating of tablets may occur. However, by containing water properly, it was confirmed that the compression pressure may be lowered, and thus, a double-layer tablet having an appropriate hardness may be manufactured without tableting failures (see Experimental Example 2).

Therefore, as the double-layer tablet contains colloidal silicon dioxide in the second layer containing metformin, tableting failure may not occur, and thus, productivity is high, and physical stability may be secured, and thus, layer separation or the like may not occur during storage. In addition, generation of related substances during storage is maintained within a standard value, and thus, the double-layer tablet may have chemical stability. Therefore, it was confirmed that the double-layer tablet may be manufactured as a composite tablet with excellent productivity and stability.

In one embodiment, the first layer may include at least one excipient selected from a diluent, a disintegrant, a binder, and a lubricant.

The diluent may be selected from the group consisting of, for example, D-mannitol, pregelatinized starch, low-substituted hydroxypropylcellulose (L-HPC), microcrystalline cellulose (MCC), sucrose, sorbitol, xylitol, glucose, and any mixtures thereof, but is not limited thereto.

The lubricant may be selected from the group consisting of glyceryl monostearate, glyceryl palmitostearate, magnesium stearate, sodium lauryl sulfate, sodium stearyl fumarate, zinc stearate, stearic acid, hydrogenated vegetable oil, polyethylene glycol, sodium benzoate, talc, and any combination thereof, but is not limited thereto. In one embodiment, the lubricant may be sodium stearyl fumarate.

In one embodiment, the diluent may be selected from the group consisting of D-mannitol, pregelatinized starch, low-substituted hydroxypropyl cellulose (L-HPC), microcrystalline cellulose, and any combination thereof.

The disintegrant may be, for example, selected from the group consisting of crospovidone, cross-linked sodium carboxymethylcellulose (cross-linked CMC Na or croscarmellose sodium), corn starch, carboxymethylcellulose calcium, sodium starch glycolate, low-substituted hydroxypropyl cellulose (L-HPC) and mixtures thereof, but is not limited thereto. In one embodiment, the disintegrant may be cross-linked sodium carboxymethylcellulose.

The binder may be selected from the group consisting of, for example, sodium carboxymethylcellulose, ethylcellulose, hydroxyethylcellulose, hydroxypropylcellulose, hydroxypropylmethylcellulose, methylcellulose, gelatin, povidone, and any mixture thereof, but is not limited thereto. In one embodiment, the binder may be cross-linked sodium carboxymethylcellulose.

In one embodiment, the first layer may include an excipient selected from microcrystalline cellulose (MCC), mannitol, pregelatinized starch, low-substituted hydroxypropylcellulose (L-HPC), crospovidone, cross-linked sodium carboxymethylcellulose (CMC Na), magnesium stearate, sodium stearyl fumarate, and any combination thereof.

In one embodiment, the second layer may include at least one excipient selected from the group consisting of a diluent, a binder, a sustained-release carrier, a lubricant, and a mixture thereof.

The diluent may be selected from the group consisting of microcrystalline cellulose, anhydrous calcium hydrogen phosphate, mannitol, sucrose, lactose, sorbitol, xylitol, glucose, hydroxypropyl cellulose, hydroxypropylmethyl cellulose, and any combination thereof, but is not limited thereto.

The binder may be selected from the group consisting of, for example, sodium carboxymethylcellulose, ethylcellulose, hydroxyethylcellulose, hydroxypropylcellulose, hydroxypropylmethylcellulose, methylcellulose, gelatin, povidone, and any mixture thereof, but is not limited thereto.

The sustained-release carrier is known in the art and may be any suitable sustained-release carrier. The sustained-release carrier may be, for example, selected from the group consisting of hydroxypropylmethylcellulose, hydroxyethylcellulose, hydroxypropylcellulose, methylcellulose, ethylcellulose, polyethylene oxide, guar gum, locust bean gum, xanthan gum, glyceryl distearate, sodium carboxymethyl cellulose, polyvinylpyrrolidone, and any combination thereof. In one embodiment, the sustained-release carrier may be a combination of hydroxypropylmethylcellulose 2208, hydroxypropylmethylcellulose 2910, and locust bean gum. The sustained-release carrier may be contained in an amount of 10 parts to 50 parts by weight, specifically about 20 parts to 40 parts by weight, based on 100 parts by weight of metformin or a pharmaceutically acceptable salt thereof.

The lubricant may be selected from the group consisting of calcium stearate, colloidal silicon dioxide (fumed silica, Aerosil), glyceryl monostearate, glyceryl palmitostearate, magnesium stearate, sodium lauryl sulfate, sodium stearyl fumarate, zinc stearate, stearic acid, hydrogenated vegetable oil, polyethylene glycol, sodium benzoate, talc, and any combination thereof, but is not limited thereto. In one embodiment, the lubricant may be magnesium stearate.

In one embodiment, when composite tablet is stored for 4 weeks under harsh conditions at 60 °C, a total content of sitagliptin related substances in the composite tablet is 0.2 wt% or less, and a content of dapagliflozin related substances in the composite tablet is 2 wt% or less.

In one embodiment, each unit dosage form of the composite tablet may include 25 mg to 100 mg of the sitagliptin as a sitagliptin free base, 5 mg to 10 mg of dapagliflozin as a free base, and 500 mg to 1,000 mg of metformin as a free base. For example, each unit dosage form of the composite tablet may include metformin as a free base of 500 mg, 750 mg, 850 mg, or 1,000 mg, sitagliptin as a free base of 50 mg, and dapagliflozin as a free base of 5 mg.

The composite tablet may be formed by tableting in the form of a double-layer tablet with dry granules containing sitagliptin and dapagliflozin as the first layer and wet granules containing metformin as the second layer.

The double-layer tablet may additionally include a film coating layer on an outer surface. The film coating layer may include any film coating agent and colorant exhibiting immediate release properties. The film coating agent may include, but is not limited to, a mixture of HPC and HPMC, or a mixture of polyvinyl alcohol (PVA) and polyethylene glycol (PEG). The colorant may include, but is not limited to, titanium dioxide, iron oxide, and the like. A typical commercially available film coating agent is Opadry^{®}. The film coating layer may serve to mask taste and provide stability to the final composite tablet.

The double-layer tablet may be used for treatment of adult patients with type 2 diabetes that may not be sufficiently controlled with sitagliptin, dapagliflozin, or metformin alone or in double combination, or patients who are already receiving triple combination therapy with sitagliptin, dapagliflozin, and metformin. The double-layer tablet may be administered once a day, twice a day, three times a day, or four times a day depending on a content of contained active ingredients.

According to another aspect, provided is a method of preparing the composite tablet according to the one aspect, the method including:
preparing a mixture portion including sitagliptin or a pharmaceutically acceptable salt thereof, or a hydrate thereof, and dapagliflozin or a pharmaceutically acceptable salt thereof, or a hydrate thereof, and an excipient;
dry-granulating the mixture portion;
preparing a first mixture portion by adding a lubricant to the obtained granules and mixing the lubricant with the granules;
preparing metformin wet granules containing metformin or a pharmaceutically acceptable salt thereof, and an excipient;
drying the obtained metformin wet granules;
preparing a second mixture portion by mixing the dried metformin wet granules with colloidal silicon dioxide and a lubricant; and
compressing the first mixture portion into a first layer and the second mixture portion into a second layer by using a double-layer tablet press.

For details of the method of preparing the composite tablet, the description of the composite tablet according to an aspect of the present disclosure may also be applied.

In preparing of the first layer, the dry-granulating may be performed according to a dry-granulation method known in the art. In one embodiment, the dry-granulating may include forming a compact using a roller compactor with a mixture including an active ingredient, a diluent, a binder, and a lubricant.

The wet-granulating in preparing of the second layer may be performed according to a wet-granulation method known in the art. In one embodiment, the drying of the obtained metformin wet granules may be performed such that a water content may be in a range of 2.5 wt% to 3.5 wt%.

Each process involved in the method of preparing the composite tablet may be performed based on a common double-layer tablet manufacturing process performed in the related art.

In one embodiment, a required compression pressure for double-layer tablet compression may be about 2,000 kN to 2,500 kN when manufacturing the double-layer tablet.

Hereinafter, the present disclosure will be described in further detail with reference to Examples. However, these examples are not intended to limit the scope of the one or more embodiments of the present disclosure.

### Experimental Example 1: Evaluation according to water amount of metformin layer

### (1) Method of preparing sample

Siagliptin, dapagliflozin, microcrystalline cellulose, mannitol, low-substituted hydroxypropyl cellulose, croscarmellose sodium, and sodium stearyl fumarate were sieved through a No. 20 sieve to crush large masses, followed by mixing. This mixture was pressed with a roller compactor to prepare slugs. Dry granules were prepared by sieving the prepared slugs through a No. 20 sieve. The final mixture portion of the first layer portion was prepared by mixing the prepared dry granules with sodium stearyl fumarate as a lubricant.

After sieving metformin, hydroxypropylmethylcellulose, and locust bean gum, wet granules were prepared with water as a binder solvent by a high-speed granulator machine. After drying the prepared granules according to the water standard with a fluidized bed dryer, the dried granules were sieved using a sizer. After sieving colloidal silicon dioxide, the resultant was mixed with the prepared granules, and similarly, vegetable magnesium stearate was sieved to prepare the final mixture portion of the second layer portion.

The first layer part and the second layer part were tableted to the appropriate hardness using a double-layer tableting press, and the outer coating was performed.

### (2) Evaluation of shrinkage and productivity

According to the sample preparation method, double-layer tablets of Examples 1, 2, 3, and 4 and Comparative Examples 1, 2, and 3 were prepared with the composition shown in Table 1 below. During the preparation, when preparing metformin granules, the amount of water in the metformin granule layer was adjusted as shown in Table 1 below by adjusting the water according to the drying time in the drying process. In addition, when tableting in a double-layer tablet tableting machine, each double-layer tablet was manufactured by tableting at a constant compression pressure of 2,000 kN.

**[Table 1]**

| | | | Example 1 | Example 2 | Example 3 | Example 4 | Comparative Example 1 | Comparative Example 2 | Comparative Example 3 |
|---|---|---|---|---|---|---|---|---|---|
| Characteristic | Process | Metformin granule layer water | 3.5 % | 3.0% | 2.5% | 2.0% | 4.0% | 1.5% | 1.0% |
| First layer portion (dry) | Dry granule | Sitagliptin Phosphate salt | 64.26 | | | | | | |
| | | Dapagliflozin L-proline | 7.80 | | | | | | |
| | | Microcrystalline cellulose | 104.84 | | | | | | |
| | | Mannitol | 55.20 | | | | | | |
| | | Low-substituted hydroxypropyl cellulose | 35.70 | | | | | | |
| | | Croscarmellose sodium | 7.40 | | | | | | |
| | | Sodium stearyl fumarate | 17.80 | | | | | | |
| | Final mixture | Sodium stearyl fumarate | 7.00 | | | | | | |
| Second layer portion (wet) | Wet granule | Metformin | 1000.00 | 1000.00 | 1000.00 | 1000.00 | 1000.00 | 1000.00 | 1000.00 |
| | | Hydroxypropylmethylcellulose 2208 | 270.00 | 270.00 | 270.00 | 270.00 | 270.00 | 270.00 | 270.00 |
| | | Hydroxypropylmethylcellulose 2910 | 8.00 | 8.00 | 8.00 | 8.00 | 8.00 | 8.00 | 8.00 |
| | | Locust bean gum | 40.00 | 40.00 | 40.00 | 40.00 | 40.00 | 40.00 | 40.00 |
| | Mixture | Colloidal silicon dioxide | 7.00 | 7.00 | 7.00 | 7.00 | 7.00 | 7.00 | 7.00 |
| | Final mixture | Vegetable magnesium stearate | 15.00 | 15.00 | 15.00 | 15.00 | 15.00 | 15.00 | 15.00 |

For the prepared tablets, the shrinkage of each layer was measured.

The shrinkage of each layer was measured by measuring a difference between a diameter of a major axis of a tablet immediately after tableting and a diameter of a major axis of the tablet after storage at 40° C for 1 hour, and then calculating the ratio of the difference to the diameter of the major axis of the tablet immediately after tableting as a percentage.

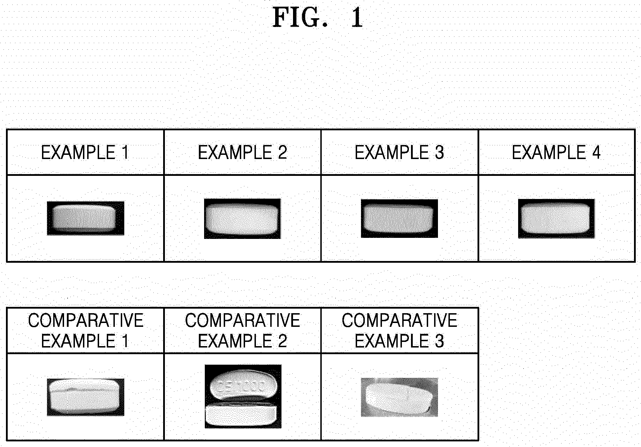

The results thereof are shown in Table 2. In addition, by observing the appearance of each tablet, hardness and friability were compared. Images of each tablet are shown in FIG. 1.

**[Table 2]**

| Shrinkage (%) | Example 1 | Example 2 | Example 3 | Example 4 | Comparative Example 1 | Comparative Example 2 | Comparative Example 3 |
|---|---|---|---|---|---|---|---|
| Upper portion | - 0.09 | - 0.08 | - 0.09 | - 0.10 | - 0.07 | Tableting - not possible | Tableting - not possible |
| Lower portion | - 0.88 | - 0.67 | - 0.53 | - 0.31 | - 1.27 | Tableting - not possible | Tableting - not possible |
| Shrinkage difference | 0.79 | 0.59 | 0.44 | 0.21 | 1.20 | Tableting - not possible | Tableting - not possible |

According to the results of Table 2 and FIG. 1, when the water in the metformin granule layer (lower layer) was 4.0 % or more (Comparative Example 1), there was no problem in productivity. However, layer separation due to the shrinkage difference between the upper and lower layers was confirmed upon coating tablets and under harsh and accelerated conditions. In addition, when the water content was less than 2.0 % (Comparative Examples 2 and 3), it was confirmed that the hardness of the tablet was not secured due to insufficient water at a constant compression pressure, and the tablet was easily broken. When the water in the metformin granule layer (lower layer) was 2.0 wt% to 3.5 wt%, sufficient hardness was secured, and no layer separation was observed.

### Experimental Example 2: Evaluation of shrinkage according to amount of colloidal silicon dioxide

Tablets of Examples 5 to 19 were prepared in the same manner as in the method in Experimental Example 1 and the prescription in Table 1, except that the amount of water and the amount of colloidal silicon dioxide in the metformin granule layer were as shown in Table 3 below and the tableting is done such that each double-layer tablet had the same hardness (20 kp). At this time, the compression pressure required for tableting each double-layer tablet to have the same hardness (20 kp) was measured, and after tableting, the appearance of each tablet were observed to check whether tableting was failed. In addition, the shrinkage difference of each of the prepared double-layer tablets was evaluated in the same manner as in Experimental Example 1.

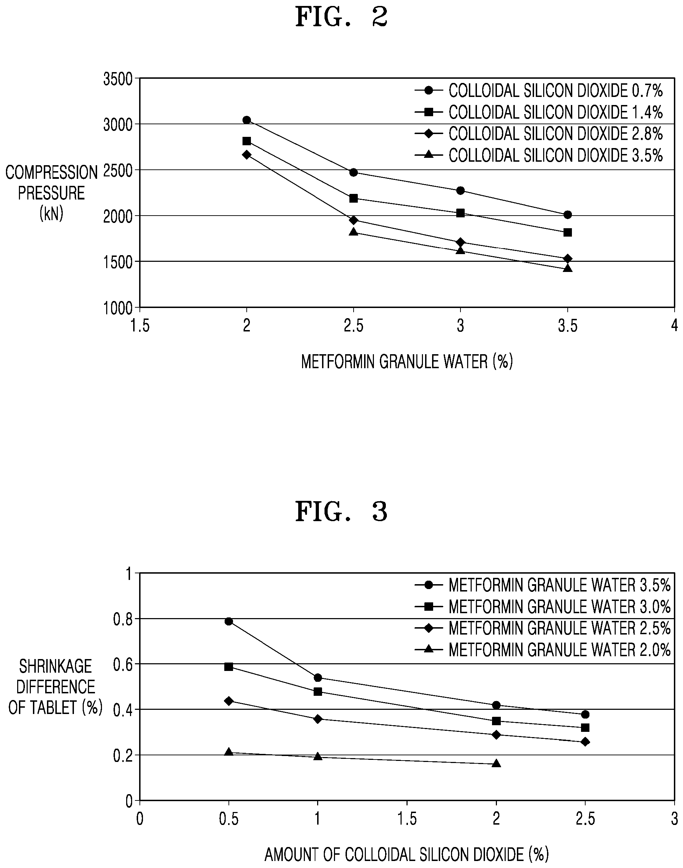

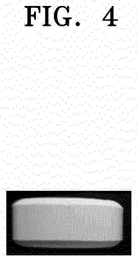

The results of measuring the required compression pressure and the shrinkage difference of the tablets for each double-layer tablet are shown in Table 4 below. In addition, based on data obtained as a result of the evaluation of the required compression pressure and the shrinkage difference of the tablets, the required compression pressure according to the water content of the metformin granule layer is shown in FIG. 2, and the shrinkage difference of the tablets according to the content of colloidal silicon dioxide is shown in FIG. 3. As a result of observing the appearance of the prepared composite tablet, smooth tablets were produced without tableting failure in the case of Examples 5 to 13 and 17 to 19 (FIG. 4 shows an image of the tablet of Example 6). Tableting failure such as capping or laminating occurred only in the case of Examples 14 to 16. According to the claims, the colloidal silicon dioxide is present at 0.7 percent by weight (wt%) to 2.8 wt% relative to active ingredients of metformin.

**[Table 3]**

| Tableting to have 20 kp hardness | Example 5 | Example 6 | Example 7 | Example 8 | Example 9 | Example 10 | Example 11 | Example 12 | Example 13 |
|---|---|---|---|---|---|---|---|---|---|
| Metformin granule water (%) | 3.5 | 3.5 | 3.5 | 3.0 | 3.0 | 3.0 | 2.5 | 2.5 | 2.5 |
| Amount of colloidal silicon dioxide (mg, % based on active ingredient) | 7.0 mg (0.7 %) | 14.0mg (1.4 %) | 28.0 mg (2.8 %) | 7.0 mg (0.7 %) | 14.0 mg (1.4 %) | 28.0 mg (2.8 %) | 7.0 mg (0.7 %) | 14.0 mg (1.4 %) | 28.0 mg (2.8 %) |

| Tableting to have 20 kp hardness | Example 14 | Example 15 | Example 16 | Example 17 | Example 18 | Example 19 | | | |
|---|---|---|---|---|---|---|---|---|---|
| Metformin granule water (%) | 2.0 | 2.0 | 2.0 | 3.5 | 3.0 | 2.5 | | | |
| Amount of colloidal silicon dioxide (mg, % based on active ingredient) | 7.0 mg (0.7 %) | 14.0 mg (1.4 %) | 28.0 mg (2.8 %) | 35.0 mg (3.5 %) | 35.0 mg (3.5 %) | 35.0 mg (3.5 %) | | | |

**[Table 4]**

| Tableting to have 20 kp hardness | Example 5 | Example 6 | Example 7 | Example 8 | Example 9 | Example 10 | Example 11 | Example 12 | Example 13 |
|---|---|---|---|---|---|---|---|---|---|
| Required compression pressure (kN) | 2010 | 1820 | 1530 | 2270 | 2030 | 1710 | 2470 | 2190 | 1950 |
| Shrinkage difference of tablet (%) | 0.79 | 0.54 | 0.42 | 0.59 | 0.48 | 0.35 | 0.44 | 0.36 | 0.29 |

| Tableting to have 20 kp hardness | Example 14 | Example 15 | Example 16 | Example 17 | Example 18 | Example 19 | | | |
|---|---|---|---|---|---|---|---|---|---|
| Required compression pressure (kN) | 3040 | 2810 | 2660 | 1420 | 1610 | 1820 | | | |
| Shrinkage difference of tablet (%) | 0.21 | 0.19 | 0.16 | 0.38 | 0.32 | 0.26 | | | |

FIG. 2 is a graph showing required compression pressures (y-axis) according to water content of the metformin granule layer (x-axis) measured from double-layer tablets including colloidal silicon dioxide of various contents and metformin granule layers of various water contents.

FIG. 3 is a graph showing shrinkage difference of tablets (y-axis) according to colloidal silicon dioxide content (x-axis) measured from double-layer tablets including colloidal silicon dioxide of various contents and metformin granule layers of various water contents.

According to the result of FIG. 2, it was found that the required compression pressure decreased as the water of the metformin granule increased to secure the same hardness of 20 kp. In addition, based on the same amount of granule water, the required compression pressure decreased as the amount of colloidal silicon dioxide increased. In addition, in the case of Examples 14, 15, and 16, the compression pressure was found to be 2,500 kN or higher. When the compression pressure was 2,500 kN or higher, tableting failures such as capping or laminating occurred due to air release in the tablet due to excessive compression pressure during the process of tableting double-layer tablet. Therefore, it was confirmed that examples with a compression pressure of 2,500 kN or less is suitable.

In addition, according to the results of FIG. 3, as the amount of colloidal silicon dioxide in each metformin granule increased, the shrinkage difference of tablet decreased.

According to the claims, the colloidal silicon dioxide is present at 0.7 percent by weight (wt%) to 2.8 wt% relative to active ingredients of metformin. According to the results of the FIGS. 2 and 3, when colloidal silicon dioxide, is included at 0.7 wt% to 3.5 wt% based on metformin active ingredient with 2.5 wt% to 3.5 wt% of water content in metformin granule, which corresponds to Examples 5 to 13 and 17 to 19, it was confirmed that there was no problem in tableting performance and shrinkage difference of tablet. In addition, according to the results of FIG. 1, the result of Experimental Example 1, in the case of Example 4, by adjusting the compression pressure to about 2,000 kN, it was confirmed that there was no problem in tableting performance and shrinkage difference of tablet (standard: 1 % or less). Therefore, while including colloidal silicon dioxide in an amount of 0.7 wt% to 3.5 wt% based on metformin active ingredient at 2.5 to 3.5 wt% of water content in metformin granule and adjusting the compression pressure to 2,500 kN or less, for example. 2,000 kN to 2,500 kN, it was evaluated that a composite tablet with no problem in tableting performance and shrinkage difference of tablet, can be obtained.

### Experimental Example 3: Evaluation of related substance according to amount of colloidal silicon dioxide

By evaluating the amounts of the related substances of sitagliptin and dapagliflozin for Examples 5 to 7 and Comparative Examples 17 to 19, the temporal stability of the composite tablet according to the amount of colloidal silicon dioxide was evaluated. Specifically, for each double-layer tablet, the total related substances with respect to each of the active ingredients were measured after 1, 2, and 4 weeks under harsh conditions of 60 °C.

The measurement method of the related substance is as follows.

### Sample preparation

5 tablets prepared in the Examples were precisely weighed. Then, the tablets were put in a 1,000 mL-volumetric flask, followed by adding a magnetic bar and about 600 mL of a diluent and stirring for 60 minutes to fully dissolve. Then, the magnetic bar was taken out, and diluent was added up to the mark. This solution was filtered through a 0.45 µm-membrane filter and used as a sample solution. The test was carried out under the following conditions.

### Mobile phase

A solution: pH 3.0 buffer solution, B solution: acetonitrile (ACN)

### Diluent

Mobile phase A solution : Mobile phase B solution = 60 : 40

### HPLC condition

column: column charged with 2.7 µm C18 phases for chromatography in a stainless pipe having an inner diameter of 4.6 mm and a length of 150 mm
pump: 0.8 mL/min
Injection volume : 10 µL
UV lamp: 220 nm
Analysis time: 80 minutes

**[Table 5]**

| **Minutes** | **A solution** | **B solution** |
|---|---|---|
| 0 | 80 | 20 |
| 5 | 80 | 20 |
| 60 | 40 | 60 |
| 65 | 80 | 20 |
| 80 | 80 | 20 |

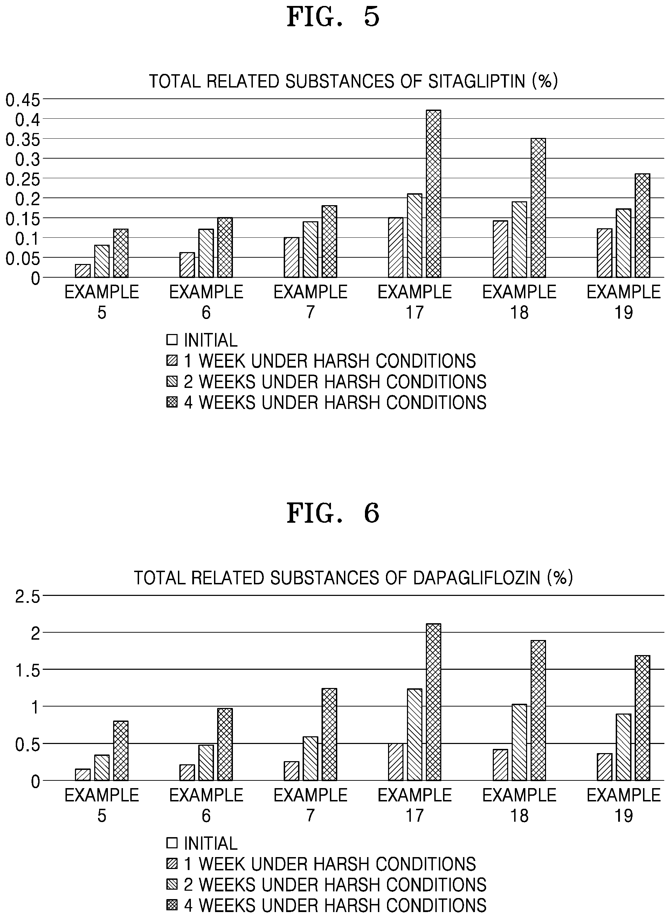

The results are shown in Tables 6 and 7. The graphs thereof are shown in FIGS. 5 and 6.

FIG. 5 is a graph showing measurement results of the total amount of related substances of sitagliptin measured over time from double-layer tablets, including colloidal silicon dioxide of various contents and metformin granule layers of various water contents under harsh conditions of 60 °C.

FIG. 6 is a graph showing measurement results of the total amount of related substances of dapagliflozin measured over time from double-layer tablets, including colloidal silicon dioxide of various contents and metformin granule layers of various water contents under harsh conditions of 60 °C.

**[Table 6]**

| Sitagliptin Total related substance (%) | Example 5 | Example 6 | Example 7 | Example 17 | Example 18 | Example 19 |
|---|---|---|---|---|---|---|
| Initiation | 0 | 0 | 0 | 0 | 0 | 0 |
| 1 week under harsh conditions | 0.03 | 0.06 | 0.1 | 0.15 | 0.14 | 0.12 |
| 2 weeks under harsh conditions | 0.08 | 0.12 | 0.14 | 0.21 | 0.19 | 0.17 |
| 4 weeks under harsh conditions | 0.12 | 0.15 | 0.18 | 0.42 | 0.35 | 0.26 |

**[Table 7]**

| Dapagliflozin Total related substance (%) | Example 5 | Example 6 | Example 7 | Example 17 | Example 18 | Example 19 |
|---|---|---|---|---|---|---|
| Initiation | 0 | 0 | 0 | 0 | 0 | 0 |
| 1 week under harsh conditions | 0.15 | 0.21 | 0.25 | 0.51 | 0.42 | 0.35 |
| 2 weeks under harsh conditions | 0.34 | 0.47 | 0.58 | 1.23 | 1.03 | 0.89 |
| 4 weeks under harsh conditions | 0.79 | 0.97 | 1.24 | 2.11 | 1.89 | 1.68 |

By applying the standards of the United States Pharmacopoeia (USP) and existing commercially available single agents, standard levels for total related compound content of sitagliptin was set to within 0.2 %, and standard levels for total related compound content of dapagliflozin was set to within 2.0 %.

According to the results of Tables 6 and 7 and FIGS. 5 and 6, in the case of Examples 5, 6, and 7 containing colloidal silicon dioxide in an amount of 2.8 wt% or less, both sitagliptin and dapagliflozin had an increased amount of the related substance within the standard up to 4 weeks under harsh conditions. In contrast, in the case of Examples 17, 18, and 19 containing colloidal silicon dioxide in an amount of 3.5 wt%, it showed an increase in the related substance exceeding the standard or close to the standard for both sitagliptin and dapagliflozin in 4 weeks under harsh conditions. Also, overall, as the amount of colloidal silicon dioxide increased, the related substances of sitagliptin and dapagliflozin increased. Therefore, it was evaluated that the amount of colloidal silicon dioxide used within 2.8 wt% may ensure stability. According to the claims, the colloidal silicon dioxide is present at 0.7 percent by weight (wt%) to 2.8 wt% relative to active ingredients of metformin.

## Claims

1. A composite tablet comprising: a first layer comprising dry granules prepared by dry-granulation that include sitagliptin or a pharmaceutically acceptable salt thereof, or a hydrate thereof, and
dapagliflozin or a pharmaceutically acceptable salt thereof, or a hydrate thereof; and
a second layer comprising wet granules prepared by wet-granulation that include metformin or a pharmaceutically acceptable salt thereof and colloidal silicon dioxide,
wherein the colloidal silicon dioxide is present at 0.7 percent by weight (wt%) to 2.8 wt% relative to active ingredients of metformin.

2. The composite tablet of claim 1, wherein a shrinkage difference between the first layer and the second layer is within 1 percent (%), wherein the shrinkage of each layer is calculated by the ratio as a percentage of the difference in the diameter of the major axis of the tablet measured before and after storage at 40°C for 1 hour following tableting.

3. The composite tablet of claim 1, wherein the metformin wet granules of the second layer have a water content of 2.5 wt% to 3.5 wt% relative to the weight of the metformin wet granules.

4. The composite tablet of claim 1, wherein the first layer contains sitagliptin phosphate and dapagliflozin L-proline as active ingredients.

5. The composite tablet of claim 1, wherein the second layer contains metformin free base or metformin hydrochloride as an active ingredient.

6. The composite tablet of claim 1, wherein the first layer comprises an excipient selected from microcrystalline cellulose (MCC), mannitol, pregelatinized starch, low-substituted hydroxypropyl cellulose (L-HPC), crospovidone, cross-linked sodium carboxymethylcellulose (CMC Na), magnesium stearate, sodium stearyl fumarate, and any combination thereof.

7. The composite tablet of claim 1, wherein the second layer comprises an excipient selected from hydroxypropylmethylcellulose, locust bean gum, microcrystalline cellulose, mannitol, sucrose, lactose, sorbitol, xylitol, glucose, colloidal silicon dioxide, magnesium stearate, and any combination thereof.

8. The composite tablet for oral administration of claim 1, wherein each unit dosage form of the composite tablet comprises 25 mg to 100 mg of the sitagliptin as a sitagliptin free base, 5 mg to 10 mg of dapagliflozin as a free base, and 500 mg to 1,000 mg of metformin as a free base.

9. A method of preparing the composite tablet for oral administration according to any one of claims 1 to 8, the method comprising:
preparing a mixture portion comprising sitagliptin or a pharmaceutically acceptable salt thereof, or a hydrate thereof, dapagliflozin or a pharmaceutically acceptable salt thereof, or a hydrate thereof, and an excipient;
dry-granulating the mixture portion; and
preparing a first mixture portion by adding a lubricant to the obtained granules and mixing the lubricant with the granules;
preparing metformin wet granules containing metformin or a pharmaceutically acceptable salt thereof, and an excipient;
drying the obtained metformin wet granules;
preparing a second mixture portion by mixing the dried metformin wet granules with colloidal silicon dioxide and a lubricant; and
compressing the first mixture portion into a first layer and the second mixture portion into a second layer, by using a double-layer tablet press.

10. The method of claim 9, wherein in preparing the first layer, the dry-granulating comprises forming a compact by using a roller compactor.

11. The method of claim 9, wherein the drying of the obtained metformin wet granules is performed such that a water content is 2.5 wt% to 3.5 wt% relative to the weight of the metformin wet granules.

12. The method of claim 9, wherein in the compressing, a required compression pressure for a double-layer compression is 2,000 kN to 2,500 kN.

## Patentansprüche

1. Verbundtablette, umfassend: eine erste Schicht, die durch Trockengranulierung hergestelltes Trockengranulat umfasst, das Sitagliptin oder ein pharmazeutisch akzeptables Salz davon oder ein Hydrat davon enthält, und
Dapagliflozin oder ein pharmazeutisch akzeptables Salz davon oder ein Hydrat davon; und
eine zweite Schicht, die durch Nassgranulierung hergestelltes Nassgranulat umfasst, das Metformin oder ein pharmazeutisch akzeptables Salz davon und kolloidales Siliziumdioxid enthält,
wobei das kolloidale Siliziumdioxid zu 0,7 Gewichtsprozent (Gew.-%) bis 2,8 Gew.-%, bezogen auf die Wirkstoffe von Metformin, vorhanden ist.

2. Verbundtablette nach Anspruch 1, wobei eine Schrumpfungsdifferenz zwischen der ersten Schicht und der zweiten Schicht kleiner or gleich 1 Prozent (%) liegt, wobei die Schrumpfung jeder Schicht durch das Verhältnis als Prozentsatz der Differenz des Durchmessers der Hauptachse der Tablette, gemessen vor und nach der Lagerung bei 40 °C für 1 Stunde nach der Tablettierung, berechnet wird.

3. Verbundtablette nach Anspruch 1, wobei das Metformin-Nassgranulat der zweiten Schicht einen Wassergehalt von 2,5 Gew.-% bis 3,5 Gew.-%, bezogen auf das Gewicht des Metformin-Nassgranulats, aufweist.

4. Verbundtablette nach Anspruch 1, wobei die erste Schicht Sitagliptinphosphat und Dapagliflozin L-Prolin als Wirkstoffe enthält.

5. Verbundtablette nach Anspruch 1, wobei die zweite Schicht Metforminfreie Base oder Metforminhydrochlorid als Wirkstoff enthält.

6. Verbundtablette nach Anspruch 1, wobei die erste Schicht einen Hilfsstoff umfasst, der aus mikrokristalliner Zellulose (MCC), Mannitol, vorgelatinisierter Stärke, niedrig substituierter Hydroxypropylcellulose (L-HPC), Crospovidon, vernetzter Natriumcarboxymethylcellulose (CMC Na), Magnesiumstearat, Natriumstearylfurmarat und jeder Kombination davon ausgewählt ist.

7. Verbundtablette nach Anspruch 1, wobei die zweite Schicht einen Hilfsstoff umfasst, der aus Hydroxypropylmethylcellulose, Johannisbrotkernmehl, mikrokristalliner Cellulose, Mannitol, Saccharose, Laktose, Sorbitol, Xylitol, Glucose, kolloidalem Siliziumdioxid, Magnesiumstearat und jeder Kombination davon ausgewählt ist.

8. Verbundtablette zur oralen Verabreichung nach Anspruch 1, wobei jede Einzeldosisform der Verbundtablette 25 mg bis 100 mg des Sitagliptins als Sitagliptin-freie Base, 5 mg bis 10 mg Dapagliflozin als freie Base und 500 mg bis 1.000 mg Metformin als freie Base umfasst.

9. Verfahren zur Herstellung der Verbundtablette zur oralen Verabreichung nach einem der Ansprüche 1 bis 8, wobei das Verfahren Folgendes umfasst:
Herstellen eines Mischungsanteils, der Sitagliptin oder ein pharmazeutisch akzeptables Salz davon oder ein Hydrat davon, Dapagliflozin oder ein pharmazeutisch akzeptables Salz davon oder ein Hydrat davon und einen Hilfsstoff umfasst;
Trockengranulieren des Mischungsanteils; und
Herstellen eines ersten Mischungsanteils durch Hinzufügen eines Schmiermittels zu dem erhaltenen Granulat und Mischen des Schmiermittels mit dem Granulat;
Herstellen von Metformin-Nassgranulat, das Metformin oder ein pharmazeutisch akzeptables Salz davon und einen Hilfsstoff enthält;
Trocknen des erhaltenen Metformin-Nassgranulats;
Herstellen eines zweiten Mischungsanteils durch Mischen des getrockneten Metformin-Nassgranulats mit kolloidalem Siliziumdioxid und einem Schmiermittel; und
Verpressen des ersten Mischungsanteils zu einer ersten Schicht und des zweiten Mischungsanteils zu einer zweiten Schicht unter Verwendung einer Doppelschicht-Tablettenpresse.

10. Verfahren nach Anspruch 9, wobei das Trockengranulieren beim Herstellen der ersten Schicht das Bilden eines Presslings unter Verwendung einer Walzenpresse umfasst.

11. Verfahren nach Anspruch 9, wobei das Trocknen des erhaltenen Metformin-Nassgranulats so durchgeführt wird, dass ein Wassergehalt von 2,5 Gew.-% bis 3,5 Gew.-%, bezogen auf das Gewicht des Metformin-Nassgranulats, beträgt.

12. Verfahren nach Anspruch 9, wobei beim Verpressen ein erforderlicher Pressdruck für ein Doppelschicht-Pressen 2.000 kN bis 2.500 kN beträgt.

## Revendications

1. Comprimé composite comportant : une première couche comportant des granulés secs préparés par granulation par voie sèche qui incluent de la sitagliptine ou un sel pharmaceutiquement acceptable de celle-ci, ou un hydrate de celle-ci, et
de la dapagliflozine ou un sel pharmaceutiquement acceptable de celle-ci, ou un hydrate de celle-ci ; et
une deuxième couche comportant des granulés humides préparés par granulation par voie humide qui incluent de la metformine ou un sel pharmaceutiquement acceptable de celle-ci et du dioxyde de silicium colloïdal,
dans lequel le dioxyde de silicium colloïdal est présent de 0,7 % en poids (% en poids) à 2,8 % en poids par rapport aux ingrédients actifs de metformine.

2. Comprimé composite selon la revendication 1, dans lequel une différence de retrait entre la première couche et la deuxième couche est inférieur ou égale à 1 pourcent (%), dans lequel le retrait de chaque couche est calculé par le rapport en pourcentage de la différence du diamètre de l'axe principal du comprimé mesuré avant et après conservation à 40°C pendant 1 heure après fabrication.

3. Comprimé composite selon la revendication 1, dans lequel les granulés humides de metformine de la deuxième couche ont une teneur en eau de 2,5 % en poids à 3,5 % en poids par rapport au poids des granulés humides de metformine.

4. Comprimé composite selon la revendication 1, dans lequel la première couche contient du phosphate de sitagliptine et de la dapagliflozine L-proline comme ingrédients actifs.

5. Comprimé composite selon la revendication 1, dans lequel la deuxième couche contient une base libre de metformine ou du chlorhydrate de metformine comme ingrédient actif.

6. Comprimé composite selon la revendication 1, dans lequel la première couche comporte un excipient choisi parmi la cellulose microcristalline (MCC), le mannitol, l'amidon prégélatinisé, l'hydroxypropylcellulose faiblement substituée (L-HPC), la crospovidone, la carboxyméthylcellulose de sodium réticulée (CMC Na), le stéarate de magnésium, le stéaryl furmarate de sodium et toute combinaison de ceux-ci.

7. Comprimé composite selon la revendication 1, dans lequel la deuxième couche comporte un excipient choisi parmi l'hydroxypropylméthylcellulose, la gomme de caroube, la cellulose microcristalline, le mannitol, le saccharose, le lactose, le sorbitol, le xylitol, le glucose, le dioxyde de silicium colloïdal, le stéarate de magnésium et toute combinaison de ceux-ci.

8. Comprimé composite pour administration orale selon la revendication 1, dans lequel chaque forme posologique unitaire du comprimé composite comporte 25 mg à 100 mg de sitagliptine en tant que base libre de sitagliptine, 5 mg à 10 mg de dapagliflozine en tant que base libre, et 500 mg à 1 000 mg de metformine en tant que base libre.

9. Procédé de préparation du comprimé composite pour administration orale selon l'une quelconque des revendications 1 à 8, le procédé comportant :
la préparation d'une partie de mélange comportant de la sitagliptine ou un sel pharmaceutiquement acceptable de celle-ci, ou un hydrate de celle-ci, de la dapagliflozine ou un sel pharmaceutiquement acceptable de celle-ci, ou un hydrate de celle-ci, et un excipient ;
la granulation par voie sèche de la partie de mélange ; et
la préparation d'une première partie de mélange par ajout d'un lubrifiant aux granulés obtenus et mélange du lubrifiant aux granulés ;
la préparation de granulés humides de metformine contenant de la metformine ou un sel pharmaceutiquement acceptable de celle-ci, et un excipient ;
le séchage des granulés humides de metformine obtenus ;
la préparation d'une deuxième partie de mélange par mélange des granulés humides de metformine séchés à du dioxyde de silicium colloïdal et à un lubrifiant ; et
la compression de la première partie de mélange en une première couche et de la deuxième partie de mélange en une deuxième couche, à l'aide d'une presse à comprimés à double couche.

10. Procédé selon la revendication 9, dans lequel lors de la préparation de la première couche, la granulation par voie sèche comporte la formation d'une pastille à l'aide d'un compacteur à rouleaux.

11. Procédé selon la revendication 9, dans lequel le séchage des granulés humides de metformine obtenus est réalisé de sorte qu'une teneur en eau soit de 2,5 % en poids à 3,5 % en poids par rapport au poids des granulés humides de metformine.

12. Procédé selon la revendication 9, dans lequel, lors de la compression, une pression de compression requise pour une compression à double couche est de 2 000 kN à 2 500 kN.
